(19) **Europäisches Patentamt**

**European Patent Office**

**Office européen des brevets**

(11) **EP 0 727 203 B1**

(12) **EUROPÄISCHE PATENTSCHRIFT**

(45) Veröffentlichungstag und Bekanntmachung des Hinweises auf die Patenterteilung:
**27.12.2000 Patentblatt 2000/52**

(51) Int. Cl.$^{7}$: **A61K 7/13**

(21) Anmeldenummer: **95116214.8**

(22) Anmeldetag: **14.10.1995**

(54) **Oxidationshaarfärbemittel**

Oxidation hair dye

Colorant d'oxydation pour cheveux

(84) Benannte Vertragsstaaten:
**DE ES FR GB IT**

(30) Priorität: **18.02.1995 DE 19505634**

(43) Veröffentlichungstag der Anmeldung:
**21.08.1996 Patentblatt 1996/34**

(73) Patentinhaber:
**Wella Aktiengesellschaft**
**64274 Darmstadt (DE)**

(72) Erfinder:
• **Mager, Herbert, Dr.**
**CH-1723 Marly (CH)**

• **Clausen, Thomas, Dr.**
**D-64665 Alsbach (DE)**
• **Balzer, Wolfgang R., Dr.**
**D-64665 Alsbach (DE)**

(56) Entgegenhaltungen:
| | |
|---|---|
| **EP-A- 0 007 537** | **EP-A- 0 400 330** |
| **EP-A- 0 684 035** | **EP-A- 0 686 389** |
| **EP-A- 0 688 558** | **EP-A- 0 705 598** |
| **EP-A- 0 722 710** | **WO-A-96/15765** |
| **DE-A- 3 627 398** | **GB-A- 2 163 154** |
| **GB-A- 2 168 727** | |

**Beschreibung**

Gegenstand der Erfindung sind saure oder neutrale Mittel zur oxidativen Färbung von Haaren mit einem Gehalt an einer Entwicklersubstanz-Kupplersubstanz-Kombination, wobei 2-(2,5-Diaminophenyl)ethanol als Entwicklersubstanz in Kombination mit bestimmten Kupplersubstanzen verwendet wird.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels. Diesen Oxidationsfarbstoffen können zur Erzielung einer breiteren Palette von verschiedenen Nuancen auch direktziehende Farbstoffe zugesetzt werden.

An Oxidationsfarbstoffe, die zur Färbung menschlicher Haare verwendet werden sollen, sind zahlreiche besondere Anforderungen gestellt. Die Farbstoffe müssen in toxikologischer und dermatologischer Hinsicht unbedenklich sowie nicht sensibilisierend sein und Färbungen in der gewünschten Intensität ermöglichen. Ferner wird für die erzielten Haarfärbungen eine gute Licht-, Dauerwell-, Säure- und Reibeechtheit gefordert.

In jedem Fall müssen solche Haarfärbungen ohne Einwirkung von Licht, Reibung und chemischen Mitteln über einen Zeitraum von mindestens vier bis sechs Wochen stabil bleiben. Außerdem ist es erforderlich, daß durch Kombination geeigneter Entwickler- und Kupplerkomponenten eine breite Palette verschiedener Farbnuancen erzeugt werden kann.

Zusätzlich zu den vorgenannten Anforderungen ergeben sich bei der oxidativen Haarfärbung im neutralen und sauren pH-Bereich eine ganze Reihe von Anforderungen, die durch die derzeit bekannten Haarfärbemittel nur teilweise oder überhaupt nicht erfüllt werden können.

So wird bei neutralen oder sauren Haarfärbemitteln das Haar weniger gequollen als bei alkalischen Haarfärbemitteln, wodurch die Penetration der Farbstoffe in das Haar, insbesondere im Bereich des nicht vorgeschädigten Haaransatzes, erheblich erschwert wird.

Weiterhin hat sich gezeigt, daß das für die oxidative Haarfärbung üblicherweise als Blaukuppler verwendete 4-(2'-Hydroxyethyl)amino-2-aminoanisol im neutralen und sauren pH-Bereich keine ausreichende Lagerbeständigkeit besitzt und daher in Oxidationshaarfärbemitteln mit neutralem oder saurem pH-Wert nicht eingesetzt werden kann.

Es ist deshalb erforderlich, einen auch im neutralen und sauren pH-Bereich largerbeständigen Blaukuppler zur Verfügung zu stellen, der gleichzeitig in physiologischer Hinsicht unbedenklich ist.

Ebenfalls hat es sich gezeigt, daß die Entwicklersubstanz 4-Amino-3-methylphenol, welche in alkalischen Haarfärbemitteln in Kombination mit der Kupplersubstanz 3-Amino-6-methylphenol zur Erzielung von Rottönen verwendet wird, im neutralen oder sauren pH-Bereich nicht den gewünschten Rotton ergibt.

Ein weiteres Problem besteht in der Praxis bezüglich des unterschiedlichen Aufziehverhaltens der Farbstoffe in Abhängigkeit von der Haarbeschaffenheit, wodurch eine ungleichmäßige Anfärbung der Haare erfolgt. Normalerweise neigen die verwendeten Farbstoffe dazu, auf geschädigte Haarpartien stärker aufzuziehen als auf ungeschädigte Haarpartien. Aus diesem Grunde werden in der Regel die durch übliche Alterungsprozesse und Umwelteinflüsse (zum Beispiel Sonnenlicht, Haarwäsche, Färbe- und Dauerwellbehandlungen) stärker geschädigten Haarspitzen intensiver gefärbt als die weniger geschädigten Haarlängen und der Haaransatz, wodurch ein unnatürliches, ungleichmäßiges und völlig unbefriedigendes Färbeergebnis erhalten wird. Dieses Problem des ungleichmäßigen Anfärbens der Haare ist bei den derzeit bekannten neutralen oder sauren Haarfärbemitteln noch stärker ausgeprägt als bei alkalischen Haarfärbemitteln.

Es bestand daher die Aufgabe, ein Oxidationshaarfärbemittel mit einem sauren oder neutralen pH-Wert zur Verfügung zu stellen, das eine gleichmäßige und farbsatte Färbung der Haare von den Haarspitzen bis zum Haaransatz über einen weiten Farbbereich ermöglicht, und gleichzeitig wenig oder gar nicht mutagen und sensibilisierend sowie physiologisch gut verträglich ist.

Zudem sollten mit diesem Haarfärbemittel sowohl Naturtöne als auch Blau- und Rottöne erzielt werden können.

Es wurde nun überraschend gefunden, daß durch ein Mittel zur oxidativen Färbung von Haaren mit einem pH-Wert von 5 bis 6,9 und einem Gehalt an einer Kombination aus 2-(2,5-Diaminophenyl)-ethanol als Entwicklersubstanz und mindestens einer Kupplersubstanz die gestellte Aufgabe in hervorragender Weise gelöst wird.

Überraschenderweise hat sich gezeigt, daß beispielsweise bei Verwendung einer Kombination aus 2-(2,5-Diaminophenyl)-ethanol und dem Kuppler 4-Chlorresorcin bei einem neutralen bis schwach sauren pH-Wert ein im Vergleich zur Färbung im alkalischen Bereich freundlicherer und natürlicher wirkender, gleichmäßiger und intensiver Naturton ohne jeden Gelbstich erhalten wird.

Ein ähnlicher überraschender und färbetechnisch vorteilhafter Effekt ergibt sich, wenn man im neutralen bis schwach sauren pH-Bereich eine Kombination. aus 2-(2,5-Diamino-phenyl)-ethanol und dem Kuppler 1-Naphthol einsetzt. Hierbei entsteht im Gegensatz zu dem erwarteten Violetton eine reine Blaufärbung, so daß die Kombination aus 2-(2,5-Diaminophenyl)-ethanol mit dem Kuppler 1-Naphthol oder dem sich ähnlich verhaltenden Kuppler 4-Hydroxyindol einen geeigneten Blaukuppler für den sauren oder neutralen Bereich darstellt.

Ebenfalls völlig überraschend wurde festgestellt, daß eine Kombination aus 2-(2,5-Diaminophenyl)-ethanol und dem Kuppler 4-(2'-Hydroxyethyl)amino-1,2-methylendioxybenzol im sauren oder neutralen pH-Bereich einen farbsatten aschigen Naturton, welcher ohne Grünstich ist, ergibt, während diese-Entwicklersubstanz/Kupplersubstanz-Kombination im alkalischen pH-Bereich einen verwaschenen GrÜnton ergibt, der fÜr die Haarfärbung wenig geeignet ist. Schließlich ergibt die Kombination aus 2-(2,5-Diaminophenyl)ethanol und dem Kuppler 2-Methylresorcin im neutralen bis schwach sauren Bereich eine wesentlich intensivere beigefarbene Färbung als sie bei Verwendung eines alkalischen pH-Wertes erzielt werden kann.

Die Verwendung von 2-(2,5-Diaminophenyl)-ethanol in Kombination mit bestimmten Kupplerverbindungen wird in der WO-A-96 157 65, EP-A-0 686 389, EP-A-0 688 558, EP-A-0 684 035, EP-A-0 705 598 und EP-A-0 722 710 beschrieben.

Der Gegenstand,der vorliegenden Anmeldung betrifft daher ein Mittel zur oxidativen Färbung von Haaren, welches durch Vermischen einer Farbträgermasse mit einem Oxidationsmittel unmittelbar vor der Anwendung hergestellt wird, einen pH-Wert von 5,0 bis 6,9 und einen Gehalt an einer Entwicklersubstanz/Kupplersubstanz Kombination aufweist, und dadurch gekennzeichnet ist, daß es als Entwicklersubstanz 2-(2,5-Diaminophenyl)-ethanol oder dessen physiologisch verträgliches, wasserlösliches Salz enthält.

Die für die Herstellung des erfindungsgemäßen Oxidationshaarfärbemittels verwendete Farbträgermasse enthält als Kupplersubstanz vorzugsweise Resorcin, 2-Methlyresorcin, 4-Chlorresorcin, 3-Aminophenol, 1-Naphthol, 4-Hydroxyindol, 3-Amino-6-methylphenol, 4-(2'-Hydroxyethyl)amino-1,2-methylendioxybenzol oder 3,4-Methylendioxyphenol, wobei diese Verbindungen alleine oder in Kombination miteinander eingesetzt werden können. Besonders bevorzugte Kupplersubstanzen sind hierbei 2-Methylresorcin, 4-Chlorresorcin, 1-Naphthol, 4-Hydroxyindol und 4-(2'-Hydroxyethyl)amino-1,2-methylendioxybenzol.

Die Entwicklersubstanzen und Kupplersubstanzen können in dem Haarfärbemittel als solche oder in Form der physiologisch verträglichen Salze, beispielsweise der Hydrochloride oder der Sulfate, enthalten sein.

Die Farbträgermasse soll das 2-(2,5-Diaminophenyl)ethanol oder dessen physiologisch verträgliches Salz in einer für die Haarfärbung ausreichenden Menge, insbesondere in einer Menge von 0,1 bis 6 Gewichtsprozent, vorzugsweise 0,1 bis 4 Gewichtsprozent, enthalten. Die Gesamtmenge der Kupplersubstanzen soll 0,1 bis 6 Gewichtsprozent, vorzugsweise 0,1 bis 4 betragen.

Aufgrund der vorteilhaften Eigenschaften des 2-(2,5-Diaminophenyl)ethanols soll diese Verbindung vorzugsweise als einzige Entwicklersubstanz in den Mitteln enthalten sein. Falls die Mittel noch andere Entwicklersubstanzen enthalten, muß jedoch gewährleistet sein, daß durch diese die erfindungsgemäßen Eigenschaften nicht nachteilig beeinflußt werden.

Neben den vorstehend genannten erfindungsgemäß verwendeten Kupplersubstanzen können zur Erzielung gewisser Farbnuancen gegebenenfalls zusätzlich noch weitere Kupplersubstanzen in geringerer Menge in der Farbträgermasse enthalten sein, sofern dadurch keine Beeinträchtigung der Eigenschaften des erfindungsgemäßen Mittels auftritt. Vorzugsweise enthält die Farbträgermasse jedoch ausschließlich eine oder mehrere der vorstehend genannten Kupplersubstanzen. Die Kupplersubstanzen können in der Farbträgermasse jeweils einzeln oder im Gemisch miteinander enthalten sein.

Die Gesamtmenge der in der hier beschriebenen Farbträgermasse enthaltenen Entwickler- und Kupplersubstanzen soll 0,1 bis 8 Gewichtsprozent, vorzugsweise 0,5 bis 4 Gewichtsprozent, betragen.

Die Entwicklersubstanz wird im allgemeinen in etwa äquimolaren Mengen bezogen auf die Kupplersubstanzen eingesetzt. Es ist jedoch nicht von Nachteil, wenn die Entwicklersubstanz in einem gewissen Überschuß oder Unterschuß vorhanden ist.

Weiterhin kann die Farbträgermasse auch direktziehende Farbstoffe, wie zum Beispiel Pikraminsäure, 2-Amino-6-chlor-4-nitrophenol, 4-(2'-Hydroxyethyl)amino-3-nitrotoluol und 4-(3'-Hydroxypropyl)amino-3-nitrophenol, enthalten, wobei die Einsatzmenge dieser Farbstoffe etwa 0,1 bis 4 Gewichtsprozent beträgt.

Selbstverständlich können die Kuppler- und Entwicklersubstanzen sowie die anderen Farbkomponenten, sofern es sich um Basen handelt, in Form der kosmetisch verträglichen Säureadditionssalze, beispielsweise als Hydrochlorid oder als Sulfat oder - sofern es sich um Verbindungen mit Phenol- oder Säuregruppen handelt - in Form der Salze mit Basen, zum Beispiel als Alkaliphenolate, eingesetzt werden.

Darüberhinaus können in der Farbträgermasse noch übliche kosmetische Zusätze, beispielsweise Antioxidantien wie Ascorbinsäure, Thioglykolsäure oder Natriumsulfit; Parfümöle; Komplexbildner; Netzmittel; Emulgatoren; Verdicker; Pflegestoffe und andere vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Oxidationshaarfärbemittel besitzt einen pH-Wert von 5 bis 6,9, welcher durch Vermischen der Farbträgermasse mit einem Oxidationsmittel eingestellt wird.

Die Farbträgermasse und das Oxidationsmittel werden hierbei im Gewichtsverhältnis von 5 zu 1 bis 1 zu 3 miteinander vermischt, wobei ein Gewichtsverhältnis von 1 zu 1 bis 1 zu 2 besonders bevorzugt ist.

Der pH-Wert der Farbträgermasse kann auch einen pH-Wert von größer/gleich 8 aufweisen, solange durch Vermischen der Farbträgermasse mit dem sauer eingestellten Oxidationsmittel ein pH-Wert des gebrauchsfertigen Oxidationshaarfärbemittels von 5 bis 6,9 eingestellt werden kann.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien, wie zum Beispiel Monoethanolamin, Triethanolamin, 2-Amino-2-methyl-1-propanol, Ammoniak, Natronlauge, Kalilauge oder Tris(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar auf.

Als Oxidationsmittel kommen hauptsächlich Wasserstoffperoxid oder dessen Additionsverbindungen an Harnstoff, Melamin oder Natriumbromat in Form einer 1 bis 12-prozentigen, vorzugsweise 6-prozentigen, wäßrigen Lösung in Betracht, wobei Wasserstoffperoxid besonders bevorzugt ist.

Man läßt das erfindungsgemäße Haarfärbemittel bei 15 bis 50 Grad Celsius etwa 10 bis 45 Minuten lang, vorzugsweise 30 Minuten lang, auf das Haar einwirken, spült sodann das Haar mit Wasser und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar, getrocknet.

Die nachfolgenden Beispiele sollen den Gegenstand näher erläutern, ohne ihn auf diese Beispiele beschränken zu wollen.

**Beispiele**

**Beispiel 1: Oxidationshaarfärbemittel**

**Farbträgermasse A1**

| | |
|---|---|
| 0,30 g | 2-(2,5-Diaminophenyl)ethanol-sulfat |
| 0,18 g | 4-Chlorresorcin |
| 0,30 g | Ascorbinsäure |
| 0,28 g | Natriumlaurylethersulfat |
| 0,22 g | Ammoniak (25%ige wäßrige Lösung) |
| 98,72 g | Wasser |
| 100,00 g | |

Der pH-Wert der Farbträgermasse wird mit verdünnter Phosphorsäure oder einer verdünnten Ammoniaklösung auf einen pH-Wert von 6,8 eingestellt.

Unmittelbar vor der Anwendung werden 20 Gramm der Farbträgermasse A1 mit 20 Gramm einer 6%igen, wäßrigen Wasserstoffperoxidlösung (pH = 6,8) vermischt und das erhaltene Oxidationshaarfärbemittel (pH = 6,8) auf gebleichte Haare aufgetragen. Nach einer Einwirkungszeit von 30 Minuten bei Raumtemperatur werden die Haare mit Wasser gespült und getrocknet.

Das so behandelte Haar ist in einem farbsatten natürlichen Blondton gefärbt.

**Beispiel 2: Oxidationshaarfärbemittel**

**Farbträgermasse A2**

| | |
|---|---|
| 0,30 g | 2-(2,5-Diaminophenyl)ethanol-sulfat |
| 0,26 g | 4-(2'-Hydroxyethyl)amino-1,2-methylendioxybenzol-hydrochlorid |
| 0,30 g | Ascorbinsäure |
| 0,28 g | Natriumlaurylethersulfat |
| 0,22 g | Ammoniak (25%ige wäßrige Lösung) |
| 98,64 g | Wasser |
| 100,00 g | |

Der pH-Wert der Farbträgermasse wird mit verdünnter Phosphorsäure oder einer verdünnten wäßrigen Ammoniaklösung auf einen pH-Wert von 6,8 eingestellt.

Unmittelbar vor der Anwendung werden 20 Gramm der Farbträgermasse A2 mit 20 Gramm einer 6%igen, wäßrigen Wasserstoffperoxidlösung (pH = 6,8) vermischt und das erhaltene Oxidationshaarfärbemittel, dessen pH-Wert gleich 6,8 ist, auf gebleichte Haare aufgetragen.

Nach einer Einwirkungszeit von 30 Minuten bei Raumtemperatur wird das Haar mit Wasser gespült und getrocknet.

Das so behandelte Haar besitzt eine glänzende, natürlich wirkende, dunkelblonde Färbung.

**Beispiel 3: Oxidationshaarfärbemittel**

**Farbträgermasse A3**

| | |
|---|---|
| 0,30 g | 2-(2,5-Diaminophenyl)ethanol-sulfat |
| 0,15 g | 2-Methylresorcin |
| 0,30 g | Ascorbinsäure |
| 0,28 g | Natriumlaurylethersulfat |
| 0,22 g | Ammoniak (25%ige wäßrige Lösung) |
| 98,75 g | Wasser |
| 100,00 g | |

Der pH-Wert der Farbträgermasse wird mit verdünnter Phosphorsäure oder einer verdünnten wäßrigen Ammoniaklösung auf einen pH-Wert von 6,8 eingestellt.

Unmittelbar vor der Anwendung werden 20 Gramm der Farbträgermasse A3 mit 20 Gramm einer 6%igen, wäßrigen Wasserstoffperoxidlösung (pH = 6,8) vermischt und das erhaltene Oxidationshaarfärbemittel (pH = 6,8) auf gebleichte Haare aufgetragen. Nach einer Einwirkungszeit von 30 Minuten bei Raumtemperatur werden die Haare mit Wasser gespült und getrocknet.

Es wird eine farbsatte beige-violette Färbung erhalten.

**Beispiel 4: Oxidationshaarfärbemittel**

**Farbträgermasse A4**

| | |
|---|---|
| 0,30 g | 2-(2,5-Diaminophenyl)ethanol-sulfat |
| 0,17 g | 1-Naphthol |
| 0,30 g | Ascorbinsäure |
| 0,28 g | Natriumlaurylethersufat |
| 0,22 g | Ammoniak (25%ige wäßrige Lösung) |

| 98,73 g | Wasser |
|---|---|
| 100,00 g | |

Unmittelbar vor der Anwendung werden 20 Gramm der Farbträgermasse A4 mit 20 Gramm einer 6%igen, wäßrigen Wasserstoffperoxidlösung (pH = 6,8) vermischt und das erhaltene Oxidationshaarfärbemittel (pH = 6,8) auf geleichte Haare aufgetragen. Nach einer Einwirkungszeit von 30 Minuten bei Raumtemperatur werden die Haare mit Wasser gespült und getrocknet.

Es wird eine farbsatte, neutrale Blaufärbung der Haare erhalten.

**Beispiel 5: Vergleichsversuche**

**Farbträgermasse A5 (cremeförmig)**

| 1,70 g | 2-(2,5-Diaminophenyl)ethanol-sulfat |
|---|---|
| 0,90 g | 4-Chlorresorcin |
| 0,10 g | 2-Methylresorcin |
| 0,03 g | 4-(2'-Hydroxyethyl)amino-3-nitrotoluol |
| 15,00 g | Cetylstearylalkohol-Natriumlaurylsulfat-Gemisch (90:10) |
| 1,30 g | Monoethanolamin |
| 0,91 g | Natriumdiglykollaurylethersulfat |
| 0,50 g | Natriumhydroxid |
| 0,20 g | Ascorbinsäure |
| 79,36 g | Wasser |
| 100,00 g | |

20 Gramm der vorstehend beschriebenen Farbträgermasse wurden mit 40 Gramm einer 2%igen Wasserstoffperoxidlösung, deren pH-Wert so eingestellt wurde, daß das erhaltene gebrauchsfertige Oxidationshaarfärbemittel eine pH-Wert von 6,9 (Beispiel 5a) beziehungsweise 9,4 (Beispiel 5b) aufweist, vermischt.

Das so erhaltene Oxidationshaarfärbemittel mit pH = 6,9 beziehungsweise mit pH = 9,4 wurde auf (I) ungeschädigtes, hellblondes Naturhaar beziehungsweise (II) durch eine Dauerwellbehandlung vorgeschädigtes, hellblondes Naturhaar aufgetragen.

Nach einer Einwirkungszeit von 30 Minuten bei 40 Grad Celsius wurde das Haar mit Wasser gespült und getrocknet.

Anschließend wurden die Farbmeßwerte mit einem Farbmeßgerät, Typ Chromameter II der Firma Minolta, bestimmt.

Das Ergebnis ist in den nachfolgenden Tabellen 1 und 2 zusammengefaßt.

Tabelle 1

| Oxidationshaarfärbemittel gemäß Beispiel 5a (erfindungsgemäß) | | | |
|---|---|---|---|
| | L | a | b |
| ungeschädigtes Haar | 19,4 | 2,4 | 5,1 |
| vorgeschädigtes Haar | 18,4 | 2,5 | 4,7 |

Tabelle 2

| Oxidationshaarfärbemittel gemäß Beispiel 5b (nicht-erfindungsgemäß) | | | |
|---|---|---|---|
| | L | a | b |
| ungeschädigtes Haar | 21,6 | 3,4 | 8,0 |
| vorgeschädigtes Haar | 17,7 | 1,8 | 3,1 |

Die vorstehenden Farbmeßwerte zeigen deutlich, daß das erfindungsgemäße Haarfärbemittel (5a) eine gleichmäßige Anfärbung der Haare ohne Farbunterschiede zwischen geschädigtem und ungeschädigtem Haar ermöglicht, während bei dem nicht-erfindungsgemäßen Haarfärbemittel ein deutlicher Unterschied zwischen den Anfärbungen auf ungeschädigtem und geschädigtem Haar zu beobachten ist.

Dieses Ergebnis wird durch den in Tabelle 3 aufgeführten Vergleich der Gesamtfarbabweichung ΔE für das erfindungsgemäße Haarfärbemittel (5a) und das nichterfindungsgemäße Haarfärbemittel (5b) bestätigt.

**Tabelle 3:    Gesamtfarbabweichung**

| Beispiel | $\Delta E = \sqrt{(\Delta L)^2 + (\Delta a)^2 + (\Delta b)^2}$ |
|---|---|
| 5a | 1,1 |
| 5b | 6,5 |

Aus den Werten für die Gesamtfarbabweichung ΔE ist klar ersichtlich, daß im Gegensatz zu dem nicht-erfindungsgemäßen Haarfärbemittel 5b bei dem erfindungsgemäße Haarfärbemittel 5a praktisch kein Unterschied zwischen den auf geschädigtem und ungeschädigtem Haar erzielten Haarfärbungen erkennbar ist.

Die vorliegenden Vergleichsversuche belegen somit eindeutig das hervorragende Farbausgleichsverhalten der erfindungsgemäßen Oxidationshaarfärbemittel.

Alle Prozentangabe stellen, soweit nicht anders angegeben, Gewichtsprozente dar.

**Patentansprüche**

1. Mittel zur oxidativen Färbung von Haaren, welches durch Vermischen einer Farbträgermasse mit einem Oxidationsmittel unmittelbar vor der Anwendung hergestellt wird, einen pH-Wert von 5 bis 6,9 und einen Gehalt an einer Entwicklersubstanz/Kupplersubstanz-Kombination aufweist, dadurch gekennzeichnet, daß es als Entwicklersubstanz 2-(2,5-Diaminophenyl)-ethanol oder dessen physiologisch verträgliches, wasserlösliches Salz enthält.

2. Mittel nach Anspruch 1, dadurch gekennzeichnet, daß die Kupplersubstanz ausgewählt ist aus Resorcin, 2-Methylresorcin, 4-Chlorresorcin, 3-Aminophenol, 1-Naphthol, 4-Hydroxyindol, 3-Amino-6-methylphenol, 4-(2'-Hydroxyethyl)amino-1,2-methylendioxybenzol und 3,4-Methylendioxyphenol.

3. Mittel nach Anspruch 1 oder 2, dadurch gekennzeichnet, daß die Entwicklersubstanz/Kupplersubstanz-Kombination in der Farbträgemasse in einer Menge von 0,1 bis 8 Gewichtsprozent enthalten ist.

4. Mittel nach einem der Ansprüche 1 bis 3, dadurch gekennzeichnet, daß die Entwicklersubstanz und die Kupplersubstanz in der Farbträgermasse jeweils in einer Menge von 0,1 bis 6 Gewichtsprozent enthalten ist.

5. Mittel nach einem der Ansprüche 1 bis 4, dadurch gekennzeichnet, daß es zusätzlich in der Farbträgermasse mindestens einen der direktziehenden Farbstoffe Pikraminsäure, 2-Amino-6-chlor-4-nitro phenol, 4-(2'-Hydroxyethyl)amino-3-nitrotoluol und 4-(3'-Hydroxypropyl)amino-3-nitrophenol enthält.

6. Mittel nach einem der Ansprüche 1 bis 5, dadurch gekennzeichnet, daß es als Antioxidantien Ascorbinsäure, Thioglykolsäure oder Natriumsulfit enthält.

7. Mittel nach einem der Ansprüche 1 bis 6, dadurch gekennzeichnet, daß es in Form einer wäßrigen oder wäßrig-alkoholischen Lösung, einer Creme, eines Geles oder einer Emulsion vorliegt.

8. Verfahren zur oxidativen Färbung von Haaren, dadurch gekennzeichnet, daß man ein Haarfärbemittel nach einem der Ansprüche 1 bis 7 in einer für die Haarfärbung ausreichenden Menge auf das Haar aufträgt, es dort 10 bis 45 Minuten bei 15 bis 50 Grad Celsius einwirken läßt, anschließend das Haar mit Wasser spült und sodann trocknet.

**Claims**

1. Agent for the oxidative colouring of hair, which is produced by mixing a dye carrier mass with an oxidation agent immediately prior to application, and which has a pH of from 5 to 6.9 and contains a developer substance/coupler substance combination, characterised in that it contains 2-(2,5-diaminophenyl)-ethanol, or the physiologically compatible, water-soluble salt thereof, as the developer substance.

2. Agent according to Claim 1, characterised in that the coupler substance is selected from resorcinol, 2-methylresorcinol, 4-chlororesorcinol, 3-aminophenol, 1-naphthol, 4-hydroxyindole, 3-amino-6-methylphenol, 4-(2'-hydroxyethyl)amino-1,2-methylene dioxy benzene and 3,4-methylene dioxy phenol.

3. Agent according to Claim 1 or 2, characterised in that the developer substance/coupler substance combination is contained in the dye carrier mass in an amount of from 0.1 to 8 weight per cent.

4. Agent according to any one of Claims 1 to 3, characterised in that the developer substance and the coupler substance are, in each case, contained in the dye carrier mass in an amount of from 0.1 to 6 weight per cent.

5. Agent according to any one of Claims 1 to 4, characterised in that it contains, additionally, at least one of the direct-acting dyes picramic acid, 2-amino-6-chloro-4-nitrophenol, 4-(2'-hydroxyethyl)amino-3-nitrotoluene and 4-(3'-hydroxypropyl)amino-3-nitrophenol.

6. Agent according to any one of Claims 1 to 5, characterised in that it contains ascorbic acid, thioglycollic acid or sodium sulphate as the antioxidant.

7. Agent according to any one of Claims 1 to 6, characterised in that it is in the form of an aqueous or aqueous-alcoholic solution, a cream, a gel or an emulsion.

8. Process for the oxidative colouring of hair, characterised in that a hair-colouring agent according to any one of Claims 1 to 7 is applied to the hair in an amount which is sufficient to colour the hair and is left there to act for 10 to 45 minutes at 15 to 50 °C, and the hair is subsequently rinsed with water and then dried.

**Revendications**

1. Agent de teinture oxydative des cheveux, préparé par mélange d'une masse de support de couleur avec un agent d'oxydation, directement avant l'application, présentant une valeur de pH allant de 5 à 6,9 et une teneur en combinaison développeur/copulateur, caractérisé en ce qu'il contient comme substance développeur du 2-(2,5-diaminophényl)-éthanol ou son sel compatible physiologiquement, soluble dans l'eau.

2. Agent selon la revendication 1, caractérisé en ce que la substance de copulateur est sélectionnée parmi la résorcine, 2-méthylrésorcine, 4-chlororésorcine, 3-aminophénol, 1-naphtol, 4-hydroxyindol, 3-amino-6-méthylphénol, 4-(2'-hydroxyéthyl)amino-1,2-méthylenedioxybenzène et 3,4-méthylenedioxyphénol.

3. Agent selon la revendication 1 ou 2, caractérisé en ce que la combinaison substance développeur/substance copulateur est contenue dans la masse de support de couleur en une quantité allant de 0,1 à 8 % en poids.

**4.** Agent selon l'une des revendications 1 à 3, caractérisé en ce que la substance développeur et la substance copulateur sont contenues respectivement dans la masse de support de couleur en une quantité allant de 0,1 à 6 % en poids.

**5.** Agent selon l'une des revendications 1 à 4, caractérisé en ce qu'il contient en plus dans la masse de support de couleur au moins l'un des colorants à application directe que sont l'acide pycraminique, le 2-amino-6-chloro-4-nitrophénol, 4-(2'-hydroxyethyl)amino-3-nitrotoluène et 4-(3'-hydroxypropyl)amino-3-nitrophénol.

**6.** Agent selon l'une des revendications 1 à 5, caractérisé en ce qu'il contient à titre d'antioxydants de l'acide ascorbique, de l'acide thioglycolique ou du sulfite de sodium.

**7.** Agent selon l'une des revendications 1 à 6, caractérisé en ce qu'il se présente sous la forme d'une solution aqueuse ou alcoolo-aqueuse, d'une crème, d'un gel ou d'une émulsion.

**8.** Procédé de teinture oxydative des cheveux, caractérisé en ce qu'on applique sur les cheveux un agent de teinture capillaire selon l'une des revendications 1 à 7, en une quantité suffisante pour obtenir la teinture capillaire, on laisse agir sur les cheveux de 10 à 45 minutes à une température de 15 à 50 °C, puis l'on rince les cheveux à l'eau et l'on sèche.